# EUROPEAN PATENT SPECIFICATION

(11) **EP 0 661 041 B1**
(45) Date of publication and mention of the grant of the patent: **10.05.2000**
(21) Application number: 94203159.2
(22) Date of filing: 31.10.1994
(51) Int. Cl.: A61K 7/16, B65D 35/24

(54) **Striped toothpaste system**
Spender für gestreifte Zahnpasta
Distributeur pour un dentifrice à rayures

(30) Priority: 05.11.1993 EP 93203102
(43) Date of publication of application: 05.07.1995
(73) Proprietor: UNILEVER N.V., 3000 DK Rotterdam (NL); UNILEVER PLC, London EC4P 4BQ (GB)
(72) Inventor: Ruefenacht, Rudolf, CH-4657 Dullinken (CH)
(74) Representative: van Gent, Jan Paulus

(56) References cited:
- EP-A- 0 076 563
- EP-A- 0 251 661
- WO-A-90/06744
- CH-A- 478 571
- US-A- 4 456 585
- US-A- 5 035 349

## Description

The present invention relates to a striping toothpaste, packaged in a tube, comprising a multilayer plastic tube with barrier properties, a nozzle with an insert extending from the discharging orifice of the tube into the interior of the tube, said insert having striping orifices, and a toothpaste formulation contained in said tube, said formulation consisting of a minor portion having a particular colour and being present in the top part of the tube, and consisting of a major portion having a different colour and being present underneath the coloured portion in the tube, said portions having a viscosity within a specific range and containing a particular binding agent.

Striping toothpastes and dispensers therefor are well-known in the art and have been on the market already for many years. Such dispensers, e.g. tubes, are e.g. described in British Patent Specification 813,514 (Marraffino). They usually consist of a tube with an insert in the discharging orifice of the tube. Such an insert usually consists of a small hollow tube, open at both ends, which tube has a plurality of longitudinal groves on its outer surface, which are connected with a small hole through the wall of the insert tube, said hole constituting the striping orifice. In general, the space of the toothpaste tube surrounding the insert tube is filled with a toothpaste of one colour, said space not extending beyond the lower end of the insert tube, and the remainder of the toothpaste tube is filled with toothpaste of another colour. Upon squeezing the filled toothpaste tube, a ribbon of the toothpaste with the other colour is extruded through the hollow insert tube, and simultaneously the toothpaste with the one colour is forced through the grooves and the small holes in the wall of the insert tube, forming small stripes of coloured toothpaste on the surface of the ribbon of the toothpaste with the other colour emerging from the discharging orifice of the toothpaste tube.

Many modifications of these inserts have been proposed, but the basic striping mechanism remains the same. Thus, the insert may be part of a nozzle which is placed over the discharging orifice of the toothpaste tube, the nozzle being externally provided with threads for receiving and engaging a screw-on closure cap.

The toothpaste tubes, used for striped toothpastes, hitherto were usually made of aluminium, or from plastic laminates containing an aluminium layer. Lately, however, toothpaste tubes are made from aluminium-free multilayer plastic materials, and while such plastic toothpaste tubes are quite suitable for ordinary toothpastes, we have found that their use for striped toothpastes causes striping problems. Due to the fact that such plastic tubes have a "memory", after squeezing the filled tube the phenomenon of suck-back occurs, by which the striped ribbon present in the top end of the insert is sucked-back due to the tube reverting into its original position after squeezing. Upon the next squeezing operation, the sucked-back striped ribbon is again extruded and new stripes are produced on said striped ribbon, which results in an unsightly , improperly striped ribbon emerging from the discharging orifice of the toothpaste tube.

We have now found that such a multilayer plastic tube, provided with a combined nozzle and insert of the type described above, can be used for packaging striping toothpastes if the portion of the toothpaste with one colour and the portion of the toothpaste with the other colour have a certain viscosity difference, and contain carboxymethylcellulose as binding agent. The difference between the viscosities of the portions we have found to have to lie between 10 and 60 Pa.s as measured in a Brookfield viscometer TE5 at 20°C.

In other words, the viscosity of the coloured portion which forms the stripes should have a viscosity which is from 10-60 Pa.s higher than the viscosity of the portion with the other colour.

Consequently, the present invention relates to a striping toothpaste, comprising two differently coloured toothpaste formulations, packaged in a tube, which upon squeezing of the tube produces extruded striped ribbons of the toothpaste, said tube comprising a plastic multilayer aluminium-free laminate tube with barrier properties, a plastic shoulder with discharging orifice attached to said multilayer tube, and a combined nozzle and striping insert placed over and into said discharging orifice, the striping insert being a hollow tube extending into the interior of the tube and having on its outer surface a plurality of longitudinal grooves connected with a small hole through the wall of said hollow tube, the space of said tube surrounding the hollow tube being filled with a toothpaste of one colour, said space not extending beyond the lower end of the hollow tube, the remaining space of the tube being filled with a toothpaste formulation with a different colour, whereby the toothpaste formulation with the one colour forming the stripes has a viscosity which is from 10-60 Pa.s higher than the viscosity of the toothpaste formulation with the different colour, both toothpaste formulations containing between 0.05 - 0.6% by weight of a carboxymethylcellulose binding agent, the viscosity being measured in a Brookfield viscometer TE5 at 20°C.

The viscosities of the portions may vary over a broad range, e.g. between 250-450 Pa.s, and they usually are between 280 and 380 Pa.s, but these ranges are not critical for the invention.

Furthermore, the formulations should contain a carboxymethylcellulose as binding agent, in an amount ranging from 0.05 -0.6% , preferably 0.1 - 0.5% by weight. Suitable types of carboxymethylcellulose binding agents are the CMC Cellulose gums ex Hercules, having a substitution degree of between 0.65 and 0.95, preferably between 0.85-0.95 such as Hercules Cellulose Gum CMC9M.

The combination of two differently coloured toothpaste formulations having viscosities as set out above and containing carboxymethylcellulose as binding agent as set out above have been found suitable for use in a plastic aluminium-free multilayer toothpaste tube with barrier properties containing a plastic shoulder attached thereto and a conbined nozzle and striping insert placed over and into the discharging orifice of the toothpaste tube to provide a striped ribbon of toothpaste upon squeezing the toothpaste tube.

In WO-A-90/06744 it is proposed that the yield values of the two differently coloured formulations should have a ratio of between 1.10:1 to 1.20:1. However, this reference also recommends that the dynamic viscosities of the formulations should not differ to any significant degree. This reference does, furthermore, not specifically indicate the necessity of using CMC as binder.

The plastic tube is made of any suitable aluminium-free multilayer plastic material with barrier properties, preferably of laminated aluminium-free multilayer plastic material made from suitable films or sheets of plastic materials to form a tube, or of any coextruded multilayer tubes or sleeves. The shoulder and discharging orifice of the tube can be made in any suitable manner from plastic material, e.g. by injection or compressing moulding, which is then sealed onto the laminated plastic tube body. The combined nozzle and insert can also be made from plastic material by injection moulding, and this combined nozzle and insert is placed over the discharging orifice of the tube such, that the hollow insert tube extends into the interior of the toothpaste tube. The dimensions of the insert and of the grooves and holes in the insert tube can vary to some extent, which is within the skill of the average expert.

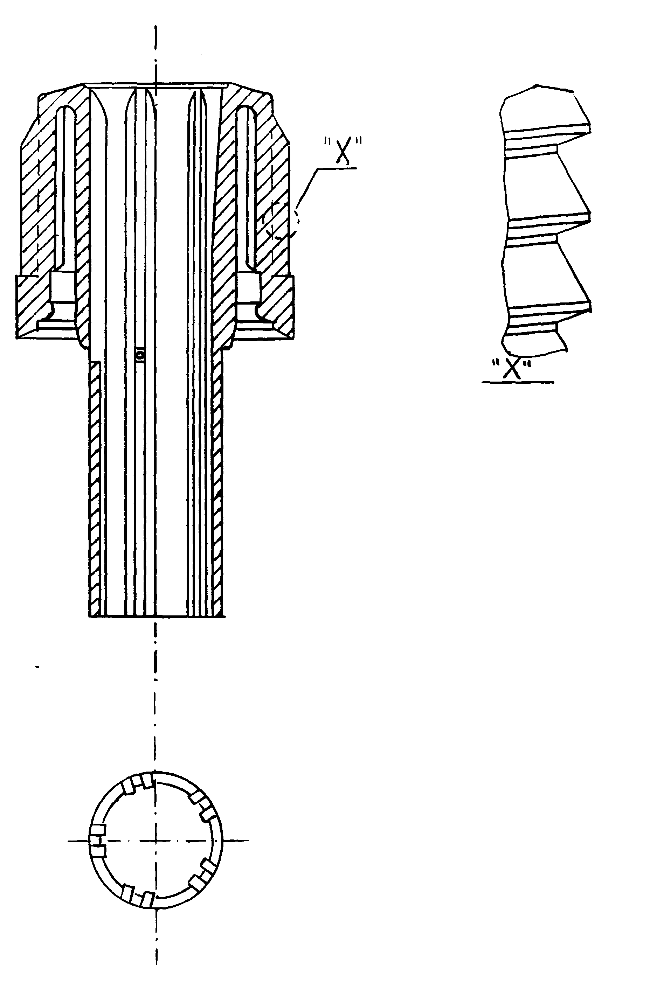

In the attached drawing, a suitable combined nozzle and insert is illustrated, which can be attached to the discharging orifice and shoulder. The nozzle is provided at its exterior with threads for a screw-on closure cap. The shoulder is sealed or welded onto the end of a coextruded plastic laminate tube.

In an example of the present invention, a coextruded multilayer plastic tube is at one end welded or sealed to a plastic injection moulded shoulder having a discharging orifice. Over said orifice a combined nozzle and insert as represented in the attached drawing is placed and a closing cap is screwed on. The tube is then inverted and first filled with a red coloured toothpaste, the amount being such that the space of the tube filled with the coloured paste does not extend over the length of the hollow insert tube. Subsequently, the tube is filled with the same toothpaste but having a different colour, e.g. white. The other end of the toothpaste tube is then sealed. Both pastes contained 0.45% of a carboxymethylcellulose binding agent. The viscosity of the red paste was about 360 Pa.s, and of the white paste about 330 Pa.s. Upon squeezing the thus filled tube, a properly striped ribbon of toothpaste was extruded, and upon the next squeeze operation no unsightly or unevenly coloured or striped ribbon was extruded, but again a properly striped ribbon was discharged. Repeating the same example, but using a white striping gel with 0.15% carboxymethylcellulose and with a viscosity of 330 Pa.s, and a blue gel as the other toothpaste portion also containing 0.15% carboxymethylcellulose and having a viscosity of 300 Pa.s also produced a properly striped ribbon of extruded toothpaste upon repeated squeezing of the toothpaste tube.

## Claims

1. A striping toothpaste, comprising two differently coloured toothpaste formulations, packaged in a tube, which upon squeezing of the tube produces extruded striped ribbons of the toothpaste, said tube comprising a plastic multilayer aluminium-free laminate tube with barrier properties, a plastic shoulder with discharging orifice attached to said multilayer tube, and a combined nozzle and striping insert placed over and into said discharging orifice, the striping insert being a hollow tube extending into the interior of the tube and having on its outer surface a plurality of longitudinal grooves connected with a small hole through the wall of said hollow tube, the space of said tube surrounding the hollow tube being filled with a toothpaste of one colour, said space not extending beyond the lower end of the hollow tube, the remaining space of the tube being filled with a toothpaste formulation with a different colour, whereby the toothpaste formulation with the one colour forming the stripes has a viscosity which is from 10-60 Pa.s higher than the viscosity of the toothpaste formulation with the different colour, both toothpaste formulations containing between 0.05 - 0.6% by weight of a carboxymethylcellulose binding agent, the viscosity being measured in a Brookfield viscometer TE5 at 20°C

2. The toothpaste according to claim 1, comprising from 0.1 - 0.5% of a carboxymethylcellulose binding agent.

3. The combination of two differently coloured toothpaste formulations having viscosities as set out in claim 1 and containing carboxymethylcellulose as binding agent as set out in claim 1, suitable for use in a plastic aluminium-free multilayer toothpaste tube with barrier properties containing a plastic shoulder attached thereto and a combined nozzle and striping insert placed over and into the discharging orifice of the toothpaste tube to provide a striped ribbon of toothpaste upon squeezing the toothpaste tube.

## Patentansprüche

1. Streifenbildende Zahnpasta, umfassend zwei verschiedenfarbige Zahnpastarezepturen, verpackt in einer Tube, die beim Zusammendrücken der Tube extrudierte gestreifte Stränge der Zahnpasta erzeugt, wobei die Tube umfasst: ein Rohr aus aluminiumfreiem mehrschichtigem Kunststofflaminat mit Sperrschichteigenschaften, eine mit dem mehrschichtigen Rohr verbundene Kunststoffschulter mit Abgabeöffnung und ein über und in die Abgabeöffnung hinein angebrachter kombinierter Düsen- und Streifenbildungseinsatz, wobei der Streifenbildungseinsatz ein hohles Rohr ist, das sich ins Innere der Tube erstreckt und auf seiner Außenseite eine Mehrzahl von Längsnuten aufweist, die mit einem kleinen Loch durch die Wand des hohlen Rohrs verbunden sind, wobei der das hohle Rohr umgebende Raum der Tube mit einer Zahnpasta von einer Farbe gefüllt ist, wobei sich der Raum nicht über das untere Ende des hohlen Rohrs hinaus erstreckt, wobei der verbleibende Raum der Tube mit einer Zahnpastarezeptur mit einer anderen Farbei gefüllt ist, wobei die Zahnpastarezeptur mit der einen Farbe, welche die Streifen bildet, eine Viskosität aufweist, die von 10 - 60 Pa.s höher ist als die Viskosität der Zahnpastarezeptur mit der anderen Farbe, wobei beide Zahnpastarezepturen zwischen 0,05 - 0,6 Gewichts-% eines Carboxymethylcellulose-Bindemittels enthalten, wobei die Viskosität bei 20°C in einem Brookfield-Viskosimeter TE5 gemessen wird.

2. Zahnpasta nach Anspruch 1, umfassend von 0,1 - 0,5 % eines Carboxymethylcellulose-Bindemittels.

3. Kombination von zwei verschiedenfarbigen Zahnpastarezepturen, die Viskositäten besitzen, wie in Patentanspruch 1 ausgeführt, und Carboxymethylcellulose als Bindemittel enthalten, wie in Patentanspruch 1 ausgeführt, geeignet zur Verwendung in einer Zahnpastatube aus aluminiumfreiem mehrschichtigem Kunststoff mit Sperrschichteigenschaften enthaltend eine damit verbundene Kunststoffschulter und einen über und in die Abgabeöffnung der Zahnpastatube hinein angebrachten kombinierten Düsen- und Streifenbildungseinsatz, um beim Zusammendrücken der Zahnpastatube für einen gestreiften Zahnpastastrang zu sorgen.

## Revendications

1. Pâte dentifrice à rayures, comportant deux formulations de pâte dentifrice différemment colorées, conditionnée dans un tube, qui, lorsque l'on appuie sur le tube, produit des rubans rayés extrudés constitués de la pâte dentifrice, ledit tube comportant un tube de stratifié multicouche en matière plastique sans aluminium, possédant des propriétés de barrière, un épaulement en matière plastique comportant un orifice de déchargement fixé sur ledit tube multicouche, et une buse et un élément d'insertion servant à former des rayures combinés placés au-dessus et à l'intérieur dudit orifice de déchargement, l'élément d'insertion étant un tube creux qui s'étend à l'intérieur du tube et possède, formées sur sa surface extérieure, une pluralité de gorges longitudinales reliées à un petit trou formé à travers la paroi dudit tube creux, l'espace existant à l'intérieur dudit tube autour du tube creux étant rempli d'une pâte dentifrice possédant une première couleur, ledit espace ne s'étendant pas au-delà de l'extrémité inférieure du tube creux, l'espace restant du tube étant rempli d'une formulation de pâte dentifrice de couleur différente, la formulation de la pâte dentifrice de la première couleur formant les rayures possédant une viscosité supérieure, à raison d'une valeur comprise entre 10 et 60 Pa.s, à la viscosité de la formulation de la pâte dentifrice possédant la couleur différente, les deux formulations de pâte dentifrice contenant entre 0,05 et 0,6 % en poids d'un agent liant composé de carboxyméthylcellulose, la viscosité étant mesurée en utilisant un viscosimètre de Brookfield TE5 à 20°C.

2. Pâte dentifrice selon la revendication 1, comportant entre 0,1 et 0,5 % d'un agent liant composé de carboxyméthylcellulose.

3. Combinaison de deux formulations de pâte dentifrice différemment colorées possédant des viscosités telles que définies dans la revendication 1 et contenant de la carboxyméthylcellulose comme agent liant.
